Europäisches Patentamt
European Patent Office
Office européen des brevets

(11) Publication number: **0 286 252 A2**

# EUROPEAN PATENT APPLICATION

(21) Application number: 88302347.5

(22) Date of filing: 17.03.88

(51) Int. Cl.4: A61K 31/42 , A61K 31/425 , A61K 31/40

(30) Priority: 17.03.87 US 26602

(43) Date of publication of application: 12.10.88 Bulletin 88/41

(84) Designated Contracting States: CH DE FR GB LI NL SE

(71) Applicant: DANA-FARBER CANCER INSTITUTE
44 Binney Street
Boston Massachusetts 02115(US)

(72) Inventor: Humphlett, Wilbert J. c/o Eastman Kodak Company
Patent Department 343 State Street
Rochester New York 14650(US)
Inventor: Chen, Lan Bo c/o Eastman Kodak Company
Patent Department 343 State Street
Rochester New York 14650(US)

(74) Representative: Nunney, Ronald Frederick Adolphe et al
Kodak Limited Patent Department Headstone Drive
Harrow Middlesex HA1 4TY(GB)

(54) **Anti-cancer composition & method featuring cyanine dyes.**

(57) There are described an anti-cancer composition and a method of manufacture of a medicament for treatment of differentiated carcinomas or melanoma, featuring adding to a suitable carrier, particular cyanine dyes.

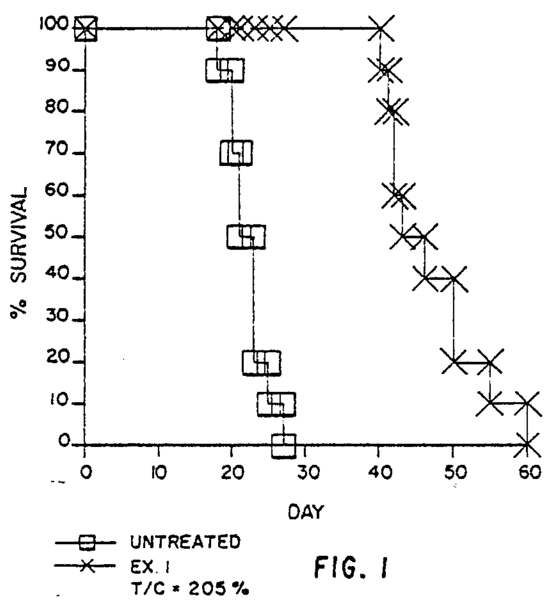


FIG. I

EP 0 286 252 A2

## ANTI-CANCER COMPOSITION & METHOD FEATURING CYANINE DYES

This invention relates to a composition and a method for treating cancers involving differentiated carcinomas or melanoma.

It does not need much repeating that cancer is a scourge of the modern world, particularly of the developed nations. According to one estimate, in such countries out of a population of 100,000, up to 20,000 people can be expected to contract cancer and fail to get effective treatment. As reported in Vol. 1 of "Dynamics and Opportunities in Cancer Management" by SRI International (1985), p. 6, about 5 million persons are likely to die of cancer in 1986. This is particularly devastating in view of the pain and incapacity which precedes actual death by cancer.

It is not surprising, therefore, that much attention is being given to finding anti-tumor agents. The need for effective anti-tumor agents is so well-known that whenever it is rumored that one has been found, the press clamors for information.

One problem with conventional chemotherapeutic drugs in the treatment of cancer has been that such drugs tend to be highly toxic against healthy cells as well as the cancer cells. As a result, they produce undesirable side effects, such as alopecia (loss of hair), emisis (nausea), nephrotoxicity, cardiotoxicity, etc. Another problem has been the relative lack of success when using even the most popular drugs. For example, "Adriamycin"TM, a known chemotherapy agent, has had only a 14.5% effective response rate in treating stomach cancer in Japan, as reported in Antibiotics Chemother., Vol. 24, pages 149-159 (1978).

Yet, so severe is the problem of cancer that people take such drugs and suffer such side-effects in the hope that the cancer will be alleviated before the side-effects become unbearable. Although there is now available some selectivity in toxicity, that is, the ability of the chemotherapeutic agent to selectively kill carcinoma cells instead of healthy cells, for most such conventional chemotherapeutic agents, that selectivity does not exceed 2 or 3 as defined, for example , by the $IC_{50}$ values in in vitro studies of human carcinomas. Such conventional selectivity of 2 or 3 is not adequate because undesirable side effects still plague the patient. Selectivity values defined by the $IC_{50}$ ratios of at least 5 to 1 are needed before the selectivity becomes significant enough to predict reduced undesirable side effects.

Therefore, prior to this invention a problem has been to provide a chemotherapeutic drug having significant selective toxicity to at least some of the cancers. Particularly this need exists in the arena of differentiated carcinomas, since as explained in Discover, March, 1986, page 37, such constitute about 85% of the cancers. Such carcinomas include cancer of the so-called "hollow" organs: the breast, colon, bladder, lung, prostate, stomach and pancreas. Colon carcinomas are particularly in need of effective chemotherapeutic agents, since at present no known drug is very effective against this cancer. (Discover, March, 1986, p. 36-46, especially p. 38.)

Cyanine dyes have been suggested heretofore as useful in treating cancer. In Japanese Kokai 79/157839, a variety of cyanine "pigments" (actually, dyes) are said to be suppressants for cancers of all types. This far-reaching claim is based solely upon tests showing that the cyanines tested inhibit markedly the internal respiration of cancer cells, implying that therefore the cancer cells will die. There is reported, however, no clonogenic test results of such cyanines to establish the $IC_{50}$ value of the dyes against any, let alone all, cancer lines, nor against a normal cell control. Nor is there in vivo data establishing whether or not any of the cyanines tested actually inhibit a particular xenograted human tumor in nude mice.

Therefore, in accord with one aspect of the invention, the above problems are addressed by a pharmaceutical composition effective to treat melanoma or carcinoma cells contained in a host mammalian body comprising a therapeutically effective amount of a cyanine dye having the following structural formula:

I

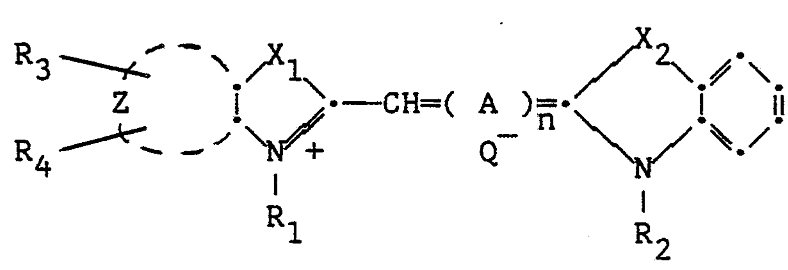

wherein $X_1$ and $X_2$ are individually O, S or Se;

Z represents the atoms necessary to complete a saturated or aromatic fused ring of six carbon ring

atoms;

A is = CH-CH = or

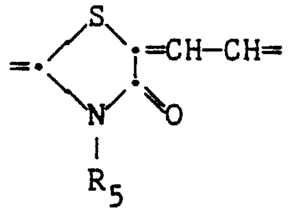

, but is the latter if $X_2$ is 0;

n is 0 or 1;

$R_1$, $R_2$ and $R_5$ are individually methyl or ethyl;

$R_3$ and $R_4$ are individually H or halide;

and Q is a pharmaceutically acceptable anion;

and a pharmaceutically suitable carrier.

In accord with another aspect of the invention, the problems of the prior art are addressed by a method of manufacture of a medicament for the treatment of differentiated carcinoma or melanoma cells in a host mammalian body,comprising adding a therapeutically effective amount of a cyanine dye having the structural formula no. I above, to a pharmaceutically suitable carrier.

Thus, it is an advantageous feature of the invention that differentiated carcinomas and melanoma are treatable in mammals using intravenous or intraperitoneal administration (hereinafter "IV" & "IP", respectively), with high selectivity.

It is a related advantageous feature of the invention that such treatment occurs while minimizing undesirable side effects.

Another advantageous feature of this invention is the provision of an anti-cancer drug effective against colon carcinoma, heretofore a cancer that has been relatively immune to chemotherapeutic treatment.

The present invention will now be described by way of example with reference to the accompanying drawings in which:

Figs. 1-8 are a plot of survival rates of a cancer in an animal model, either treated as per the invention or untreated. In these plots, "% survival" means the % of the total group (treated "T" or control "C") still surviving on the day in question. The 50% data point is selected for the T/C calculation, as being the point that is statistically meaningful.

As used herein "differentiated carcinomas" includes adenocarcinomas, which type includes about 90% of the prostate cancer that occur in about 50% of the United States male population over 65; most squamous cell carcinomas; and all transitional cell carcinomas. The term does not include oat cell carcinoma or large cell carcinoma in the lung, or poorly differentiated carcinomas. Such carcinomas are not believed to be affected by this invention.

The effective treatment of differentiated carcinomas includes the aiding of regression and palliation of tumors, inhibition of growth and remission of tumors.

Thus, the kind of cancers treatable by this invention include carcinomas from all organs, including the lung (except for those noted above), colon, breast, bladder, prostate, pancreas, stomach, vagina, esophagus, tongue, nasopharynx, liver, ovary, and testes.

The invention features the use of the cyanine dyes having the structural formula No. I. Useful dyes within this class include

3-methyl-2-[3-(3-methyl-4-benzothiazolin-2-ylidene)-1-propen-1-yl]benzothiazolium iodide; 3-ethyl-2-[3-(3-ethyl-4-benzothiazolin-2-ylidene)-1-propen-1-yl]-benzothiazolium iodide; 5-chloro-3-ethyl-2-[3-(3-ethyl-4-benzothiazolin-2-ylidene)-1-propen-1-yl]benzoselenazolium iodide; 3-ethyl-2-{3-ethyl-5-[2-(3-ethyl-4-benzoxazolin-2-ylidene)ethylidene]-4-oxo-2-thiazolidinylidenemethyl}-4,5,6,7-tetrahydrobenzothiazolium iodide; 5-chloro-3-methyl-2-[3-(3-methyl-4-benzothiazolin-2-ylidene)-1-propen-1-yl]benzothiazolium iodide; 5-chloro-3-ethyl-2-{3-ethyl-5-[2-(3-ethyl-4-benzoxazolin-2-ylidene)ethylidene]-4-oxo-2-thiazolidinylidenemethyl}-4,5,6,7-tetrahydrobenzothiazolium iodide; 3-methyl-2-(3-methyl-4-benzothiazolin-2-ylidenemethyl)benzoselenazolium iodide; 5-chloro-3-methyl-2-(3-methyl-4-benzothiazolin-2-ylidenemethyl)-benzothiazolium iodide; and 5-chloro-3-ethyl-2-(3-ethyl-4-benzoxazolin-2-ylidenemethyl)-4,5,6,7-tetrahydrobenzothiazolium iodide. Still others include all of those above, but with any of the counterions listed hereinafter in place of the iodide.

Highly preferred are the following:

3-methyl-2-[3-methyl-4-benzothiazolin-2-ylidene)-1-propen-1-yl]benzothiazolium chloride; 3-ethyl-2-[3-(3-ethyl-4-benzothiazolin-2-ylidene)-1-propen-1-yl]-benzothiazolium iodide; 5-chloro-3-ethyl-2-[3-(3-ethyl-4-benzothiazolin-2-ylidene)-1-propen-1-yl]benzoselenazolium iodide; and 3-ethyl-2-{3-ethyl-5-[2-(3-ethyl-4-benzoxazolin-2-ylidene)ethylidene-4-oxo-2-thiazolidinylidenemethyl}-4,5,6,7-tetrahydrobenzothiazolium iodide.

Any anion can be used with the cyanines of this invention, provided they are pharmaceutically acceptable. As used herein, "pharmaceutically acceptable anion" means, one that is non-toxic to the host mammal and provides an aqueous solubility to the dye of at least 0.1 mg/ml. Examples include halide such as chloride, bromide, iodide, and fluoride; sulfonate including aliphatic and aromatic sulfonate such as methanesulfonate, trifluoromethanesulfonate, p-toluenesulfonate (tosylate), naphthalene sulfonate, and 2-hydroxyethanesulfonate; sulfamate such as cyclohexanesulfamate; sulfate such as methyl sulfate and ethyl sulfate; bisulfate; borate; alkyl and dialkyl phosphate such as dimethyl phosphate, diethyl phosphate, and methylhydrogen phosphate; pyrophosphate such as trimethylpyrophosphate and diethyl hydrogen pyrophosphate; and carboxylate, preferably carboxy-and hydroxy-substituted carboxylate. Examples of such carboxylates include acetate, propionate, valerate, citrate, maleate, fumarate, lactate, succinate, tartrate and benzoate.

Because they impart to the dye greater water solubility than do many other anions, the most preferred anions are the halides.

Both the cyanines of this invention and the processes for making them are well-known per se.

Methods of delivery of the dye include implanted drug pump, intraperitoneal (IP), intravenous (IV) or intravesical injection, using a pharmaceutically acceptable carrier solvent. Such IP delivery methods are particularly useful in treating carcinomas metastasized to the peritoneal cavity. For example, ovarian carcinoma is now widely treated by IP injection.

The pharmaceutically acceptable carrier can be any carrier, such as a solvent that will sufficiently dissolve the cyanine dye. Examples of a suitable carrier solvent for human usage are a 5% dextrose solution in water, or a mixture of ethanol and a polyol such as polyethoxylated castor oil, available from the National Cancer Institute as "Diluent No. 12". Still other acceptable carrier solvents include, isotonic saline for IV and IP injections, dimethyl sulfoxide (DMSO) for intravesical injection, and in some cases, just water. However, plain water is not currently preferred.

Still other carriers that are useful include the following:

Materials such as gelatin, natural sugars such as sucrose or lactose, lecithin, pectin, starch (for example cornstarch), alginic acid, tylose, talc, lycopodium, silica (for example colloidal silica), glucose, cellulose, cellulose derivatives, for example cellulose ethers in which the cellulose hydroxyl groups are partially etherified with lower aliphatic alcohols and/or lower saturated oxyalcohols (for example, methyl hydroxypropyl cellulose, methyl cellulose, hydroxyethyl cellulose), stearates, e.g. methyl stearate and glyceryl stearate, magnesium and calcium salts of fatty acids with 12 to 22 carbon atoms, especially saturated acids (for example, calcium stearate, calcium laurate, magnesium oleate, calcium palmitate, calcium behenate and magnesium stearate), emulsifiers, oils and fats, especially of plant origin (for example, peanut oil, castor oil, olive oil, sesame oil, cottonseed oil, corn oil, wheat germ oil, sunflower seed oil, cod-liver oil), mono-, di-, and triglycerides of saturated fatty acids ($C_{12}H_{24}O_2$ to $C_{18}H_{36}O_2$ and their mixtures), e.g., glyceryl monostearate, glyceryl distearate, glyceryl tristearate, glyceryl trilaurate), pharmaceutically compatible mono-, or polyvalent alcohols and polyglycols such as glycerine, mannitol, sorbitol, pentaerythritol, ethyl alcohol, diethylene glycol, triethylene glycol, ethylene glycol, propylene glycol, dipropylene glycol, polyethylene glycol 400, and other polyethylene glycols, as well as derivatives of such alcohols and polyglycols, esters of saturated and unsaturated fatty acids (2 to 22 carbon atoms, especially 10 to 18 carbon atoms), with monohydric aliphatic alcohols (1 to 20 carbon atom alkanols), or polyhydric alcohols such as glycols, glycerine, diethylene glycol, pentaerythritol, sorbitol, mannitol, ethyl alcohol, butyl alcohol, octadecyl alcohol, etc., e.g. glyceryl stearate, glyceryl palmitate, glycol distearate, glycol dilaurate, glycol diacetate, monoacetin, triacetin, glyceryl oleate, ethylene glycol stearate, esters of polyvalent alcohols that are etherified, benzyl benzoate, dioxolane, glycerin formal, tetrahydrofurfuryl alcohol, polyglycol ethers of 1 to 12 carbon atom alcohols, dimethyl acetamine, lactamide lactates, e.g., ethyl lactate, ethyl carbonate, silicones (especially medium viscosity dimethyl polysiloxane), magnesium carbonate and the like.

Still other additives, and methods of preparation of the composition, can be found in the extant literature, for example, U.S. Patent No. 4,598,091 issued on 7/1/86.

Based on the results obtained in the animal models described hereinafter, it is estimated that the dosages of the active cyanine ingredient, for treatment of humans, would be: IV injection of 50 to 100 mg per square meter every three weeks, 0.5 to 1 mg/kg daily for 2 to 6 days, or 20 to 30 mg per square meter daily for 3 days or once weekly for 6 weeks. (The expression "per square meter" refers to the surface area

of the patient's body, as is customary.) This estimate has been made by the techniques described in Goodman & Gilman's "The Pharmacological Basis of Therapeutics" (6th edition), pages 1675-1737 (MacMillan Publishing Company, New York, 1980).

As noted above, one of the advantages of the invention is that the cyanines described herein selectively attack the cancer cells as opposed to normal cells. Although knowledge of the mechanism is not essential to the practice of the invention, it is believed this occurs through the preferential inhibition of oxygen consumption in the mitochondria of the cancer cells. Such selectivity is further shown in clonogenic in vitro studies carried out in conventional fashion. That is, cell cultures of CX-1 (human colon carcinoma) and CV-1 (normal monkey epithelial cells) are prepared and treated at various concentrations, in the conventional manner, and the concentration of the dye that inhibits 50% of the cell colony formation is denoted as $IC_{50}$. This $IC_{50}$ value is compared for both the CX-1 and CV-1 lines; that is, the value for CV-1 is divided by the value for CX-1. If the value is 10 µg/ml for CV-1, and 1 µg/ml for CX-1, then the dye is 10 times as effective in killing off the colon carcinoma cells, compared to the monkey epithelial cells. This is expressed as a selectivity "S" of 10. That is, in order to inhibit 50% of the normal cells, the drug requires ten times the concentration that inhibits 50% of the cancer cells, suggesting that the drug preferentially kills cancer cells.

The selectivity values, and the CX-1 values for four of the cyanines of this invention obtained and tested under regular lighting conditions of laboratories, are listed below in Table I, along with a fifth cyanine used for comparative purposes.

## Examples

In the examples that follow, "nude mice protocol" refers to conventional xenografted human carcinoma or melanoma cells implanted into athymic mice. It is established that effectiveness against human cancer is suggested by tests in the nude mouse-human xenograft model. This has been reported in, e.g., the Journal of the National Cancer Institute, Vol. 74, No. 4, p. 899-903, especially p. 889 (April 1985).

The following are the cyanines which have been tested in animal model studies:

Table I

| Dye No. | Cyanine | In Vitro Data CX-1 Value | S |
|---|---|---|---|
| 1 | 3-methyl-2-[3-(3-methyl-4-benzothiazolin-2-ylidene)-1-propen-1-yl]-benzothiazolium chloride | .005 µg/ml | 100 |
| 2 | 3-ethyl-2-[3-(3-ethyl-4-benzothiazolin-2-ylidene)-1-propen-1-yl]-benzothiazolium iodide. | .005 µg/ml | 80 |
| 3 | 5-chloro-3-ethyl-2-[3-(3-ethyl-4-benzothiazolin-2-ylidene)-1-propen-1-yl]benzoselenazolium iodide | .004 µg/ml | 250 |
| 4 | 3-ethyl-2-{3-ethyl-5-[2-(3-ethyl-4-benzoxazolin-2-ylidene)ethylidene]-4-oxo-2-thiazolidinylidenemethyl}-4,5,6,7-tetrahydrobenzothiazolium iodide | .050 µg/ml | 100 |
| 5 | 3-propyl-2-[3-(3-propyl-4-benzothiazolin-2-ylidene)-1-propen-1-yl]benzothiazolium iodide (Not included in the invention) | .009 µg/ml | 22 |

Examples 1-4 - LOX Melanoma Tests Using Nude Mice Protocol

A human melanoma cell line, LOX, was utilized that grows rapidly when innoculated into the peritoneal cavity of athymic mice. Tumors which had been passaged subcutaneously in athymic mice were excised under sterile conditions and converted to a single cell suspension using a metal grid with a 4 mm mesh. Red blood cells were then lysed by incubation with 0.17 molar ammonium chloride at 4°C for 20 minutes. Cells were then scored for viability with trypan blue and 5 million viable cells made up in 0.1 ml of Dulbecco modified Eagles' medium (DME) were injected intraperitoneally into a group of athymic Swiss nu/nu mice (day 0). The mice were then randomly allocated into treatment and control groups. Treatment, by intraperitoneal injection, commenced the following day (day 1). Mice received dye No. 1 or Dye No. 2 at 1 mg/kg daily for five days followed by 1.5 mg/kg on day 8, 10, 12, 14 and 16, and twice a week at 2 mg/kg thereafter. There were 10 mice in each of the treatment groups and control group. The control group received 5% dextrose solution equal in volume to that of the Dye No. 1 treated group.

In the same experiment, the comparative example 1 was administered to 10 mice using the following schedule: 0.2 mg/kg daily for five days followed by 0.5 mg/kg on day 8, 10, 12, 14 and 16, and twice a week at 1 mg/kg thereafter. (A lower dosage of this dye is required because of its toxicity. That is, attempts to use the dosage of Dye No. 1 for this Comparative Example, killed the mice before the 50% survival point of the control could be reached, clearly indicating that a lower dosage was needed.)

The purpose of this experiment was to show the importance of the length of $R_1$ and $R_2$ in the structure set forth in the Summary above. That is, "Comp. Ex. 1" differs from Ex. 2 only in that $R_1$ and $R_2$ are propyl instead of ethyl. This is the compound of interest that appears in the above-noted Japanese application 79/157839, Ex. 3.

The results of these tests appear in Table II. T/C is the ratio, expressed as a percentage, of mean survival age of the treated group, to the mean survival age of the untreated control group.

Table II

| Ex. No. | Dye No. | T/C |
|---|---|---|
| 1 | 1 | 205% |
| 2 | 2 | 176% |
| 3 | 3 | 168% |
| 4 | 4 | 166% |
| Comp. Ex. 1 | 5 | 128% |

(The results of Ex. 1 and 2 are illustrated in Figs. 1 and 2, and of Comp. Ex.1 in Fig. 3.) Any T/C value over 145% is generally considered by those skilled in the art to be indicative of increased rate of survival, or remission of tumor. Thus, Ex. 1-4 all showed successful treatment of the melanoma. However, Comp. Ex. 1 was a failure, due to $R_1$ and $R_2$ of the structure shown in the Summary, being propyl instead of ethyl or methyl. The reason for this is not very well understood, but is believed to be due in part to the stereo-specific effects that occur when $R_1$ and $R_2$ are too long.

Examples 5-6 - Ovarian Carcinoma Tests Using Nude Mice Protocol

Dye Nos. 1 and 2 were repeated, except the cancer that was treated was OVCAR -3, a human ovarian carcinoma cell line of which ten million cells were injected IP into athymic Swiss nu/nu mice. For Ex. 5, 10 mice received dye 1 by IP injection commenced the day after the cancer injection, and repeated daily at 1.5 mg/kg for four days followed by 2 mg/kg on day 7, 10, 13, 16, 19 and 21, and twice a week at 2 mg/kg thereafter. Ten mice having such injected cancer were selected as a control group and received an equal volume of 5% dextrose as that of the treated group.

For Ex. 6, 10 mice received dye no. 2 by IP injection commenced the day after cancer injection, and repeated daily for four days at 1.5 mg/kg followed by 2 mg/kg on day 7, 10, 13, 16, 19 and 21, and twice a week at 2 mg/kg thereafter. The same control group in Ex. 5 was used. For Comp. Ex. 2, the test of Comp.

Ex. 1 was repeated, this time against the ovarian carcinoma xenografts. The dye was administered daily for four days at 0.2 mg/kg, followed by 0.5 mg/kg on day 7, 10, 13, 16, 19 and 21 and twice a week at 0.5 mg/kg thereafter.

The results appear in Table III and Figs. 4-5.

Table III

| Ex. | Dye | T/C |
|---|---|---|
| 5 | 1 | 238% |
| 6 | 2 | 157% |
| Comp. Ex. 2 | 5 | 138% |

Examples 7-8: Treatment of Mouse Bladder Carcinoma Using Cyanine Dyes Administered Intraperitioneally

MB49 tumor cells, that is, mouse carcinoma cells induced by the carcinogen 7,12-dimethylbenz[a]-anthracene, were injected intraperitoneally into normal male BDF1 mice (2 X $10^6$/mouse on day 0). Cyanine Dye No. 1 or No. 2, was administered intraperitioneally in the following manner: 0.2 mg/kg on day 1, 0.4 mg/kg on day 2, 0.8 mg/kg on day 3, 1.2 mg/kg on day 4, 1.5 mg/kg on days 8, 12, 16, 20, 24. (No further injection was used thereafter). For dye No. 5: 0.1 mg/kg on day 1, 0.2 mg/kg on day 2, 0.3 mg/kg on day 3, 0.4 mg/kg on day 4, 0.5 mg/kg on day 8, 12, 16, 20, 24. (No further injection was used thereafter.) The control group of 10 mice was treated with equal volume of 5% dextrose as those treated with dye no. 1. The survival rate was calculated as in the previous examples. The results appear in Table IV.

Table IV

| Ex. | Dye No. | T/C |
|---|---|---|
| 7 | 1 | 250% |
| 8 | 2 | 190% |
| Comp. Ex. 3 | 5 | 113% |

These examples appear in Figs. 6-8, respectively. As in the case of the other cancers tested, Dye 5, the propyl analog of Dyes 1 and 2, was a failure.

Additional Comparative Examples

The following cyanines were not considered acceptable, because of their selectivity "S" values in the in vitro tests discussed above, being less than 60. Their generic structure is as follows:

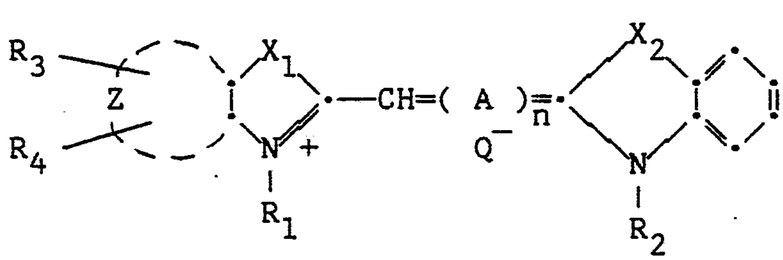

The selected groups appear in Table V along with the selectivity S values.

Table V

| Comp. Ex. | $X_1$ | $X_2$ | Group Formed by Z | A | $R_1,R_2$ | $R_3,R_4$ | Q | S |
|---|---|---|---|---|---|---|---|---|
| 4 | O | S | benzo | $=CH-CH=$ | $-CH_2CH_3$,<br>$-(CH_2)_2CHO$ | H, H | I | 50 |
| 5 | S | $C(CH_3)_2$ | cyclo-hexano | $=CH-CH=$ | $-CH_3$,<br>$-CH_3$ | together produced a fused phenyl | perchlorate | 40 |
| 6* | S | S | benzo | $SCH_3$<br>$=C-CH=$ | $-CH_3$,<br>$-CH_2CH_3$ | H, H | I | 37.5 |
| 7 | O | S | benzo | $CH_2CH_3$<br>$=C-CH=$ | $-CH_2CH_3$,<br>$-CH_2CH_3$ | H, H | I | 33.3 |
| 8** | S | Se | benzo | " | " | H, Cl | I | 33.3 |
| 9 | S | O | benzo | $CH_3$<br>$=C-CH=$ | " | together produced a fused phenyl | I | 30 |
| 10 | S | S | benzo | $=CH-CH=_2$ | $-(CH_2)_2OH$,<br>$-(CH_2)_2OH$ | H, H | Br | 26.7 |

* This was tested also in a nude mice experiment against LOX Melanoma and found to produce a T/C of 91%.

** Compare this with dye No. 3, Table I.

## Claims

1. A pharmaceutical composition effective to treat melanoma or differentiated carcinoma cells contained in a host mammalian body comprising

a therapeutically effective amount of a cyanine dye having the following structural formula:

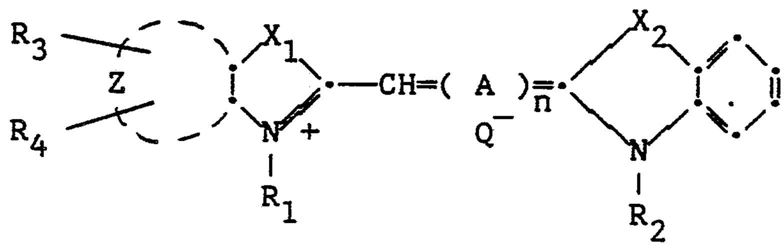

wherein $X_1$ and $X_2$ are individually O, S or Se;

Z represents the atoms necessary to complete a saturated or aromatic fused ring of six carbon ring atoms;

A is = CH-CH = or

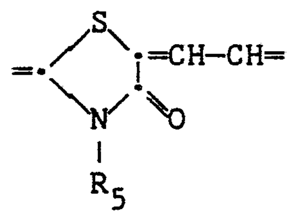

, but is the latter if $X_2$ is 0;

n is 0 or 1;

$R_1$, $R_2$ and $R_5$ are individually methyl or ethyl;

$R_3$ and $R_4$ are individually H or halide;

and Q is a pharmaceutically acceptable anion;

and a pharmaceutically suitable carrier.

2. A composition defined in claim 1, wherein said dye is 3-methyl-2-[3-(3-methyl-4-benzothiazolin-2-ylidene)-1-propen-1-yl]-benzothiazolium chloride.

3. A composition as defined in claim 1, wherein said dye is 3-ethyl-2-[3-(3-ethyl-4-benzothiazolin-2-ylidene)-1-propen-1-yl]benzothiazolium chloride.

4. A composition as defined in claim 1, wherein said dye is 5-chloro-3-ethyl-2-[3-(3-ethyl-4-benzothiazolin-2-ylidene)-1-propen-1-yl]benzoselenazolium iodide.

5. A composition as defined in claim 1, wherein said dye is 3-ethyl-2-{3-ethyl-5-[2-(3-ethyl-4-benzoxazolin-2-ylidene)ethylidene]-4-oxo-2-thiazolidinylidenemethyl}-4, 5,6,7-tetrahydrobenzothiazolium iodide.

6. A composition as defined in claim 1, wherein said effective amount is 50 to 100 mg/m$^2$ every three weeks, 0.5 to 1 mg/kg daily for 2 to 6 days, or 20 to 30 mg/m$^2$ daily for 3 days or once weekly for 6 weeks, administered by IV.

7. A method of manufacture of a medicament for the treatment of differentiated carcinoma or melanoma cells in a host mammalian body, comprising adding a therapeutically effective amount of a cyanine dye having the following structural formula:

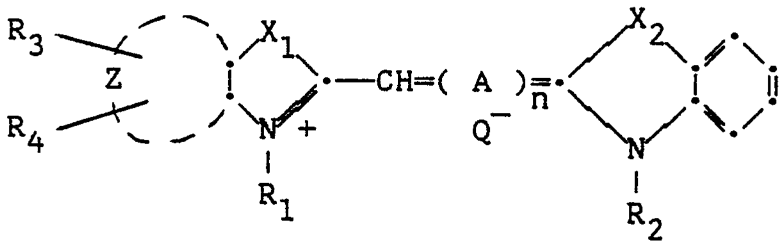

wherein $X_1$ and $X_2$ are individually O, S or Se;

Z represents the atoms necessary to complete a saturated or aromatic fused ring of six carbon ring atoms;

A is = CH-CH = or

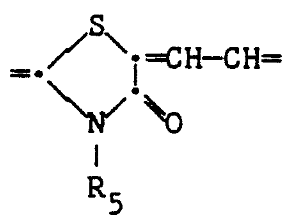

, but is the latter if $X_2$ is 0;

n is 0 or 1;

$R_1$, $R_2$ and $R_5$ are individually methyl or ethyl;

$R_3$ and $R_4$ are individually H or halide;

and Q is a pharmaceutically acceptable anion;

to a pharmaceutically suitable carrier.

8. A method as defined in claim 7 wherein said dye is 3-methyl-2-[3-(3-methyl-4-benzothiazolin-2-ylidene)-1-propen-1-yl]benzothiazolium chloride.

9. A method as defined in claim 7, wherein said dye is 3-ethyl-2-[3-(3-ethyl-4-benzothiazolin-2-ylidene)-1-propen-1-yl]benzothiazolium chloride.

10. A method as defined in claim 7, wherein said dye is 5-chloro-3-ethyl-2-[3-(3-ethyl-4-benzothiazolin-2-ylidene)-1-propen-1-yl]benzoselenazolium iodide.

11. A method as defined in claim 7, wherein said dye is 3-ethyl-2-{3-ethyl-5-[2-(3-ethyl-4-benzoxazolin-2-ylidene)ethylidene]-4-oxo-2-thiazolidinylidenemethyl}-4,5,6,7-tertahydrobenzothiazolium iodide.

12. A method as defined in claim 7, wherein said effective amount is 50 to 100 mg/m$^2$ every three weeks, 0.5 to 1 mg/kg daily for 2 to 6 days, or 20 to 30 mg/m$^2$ daily for 3 days or once weekly for 6 weeks, administered by IV.

13. A method as defined in claim 7, wherein said carcinoma is selected from the group consisting of carcinoma of the lung, colon, breast, bladder, prostate, pancreas, stomach, vagina, esophagus, tongue, nasopharynx, liver, ovary and testes.

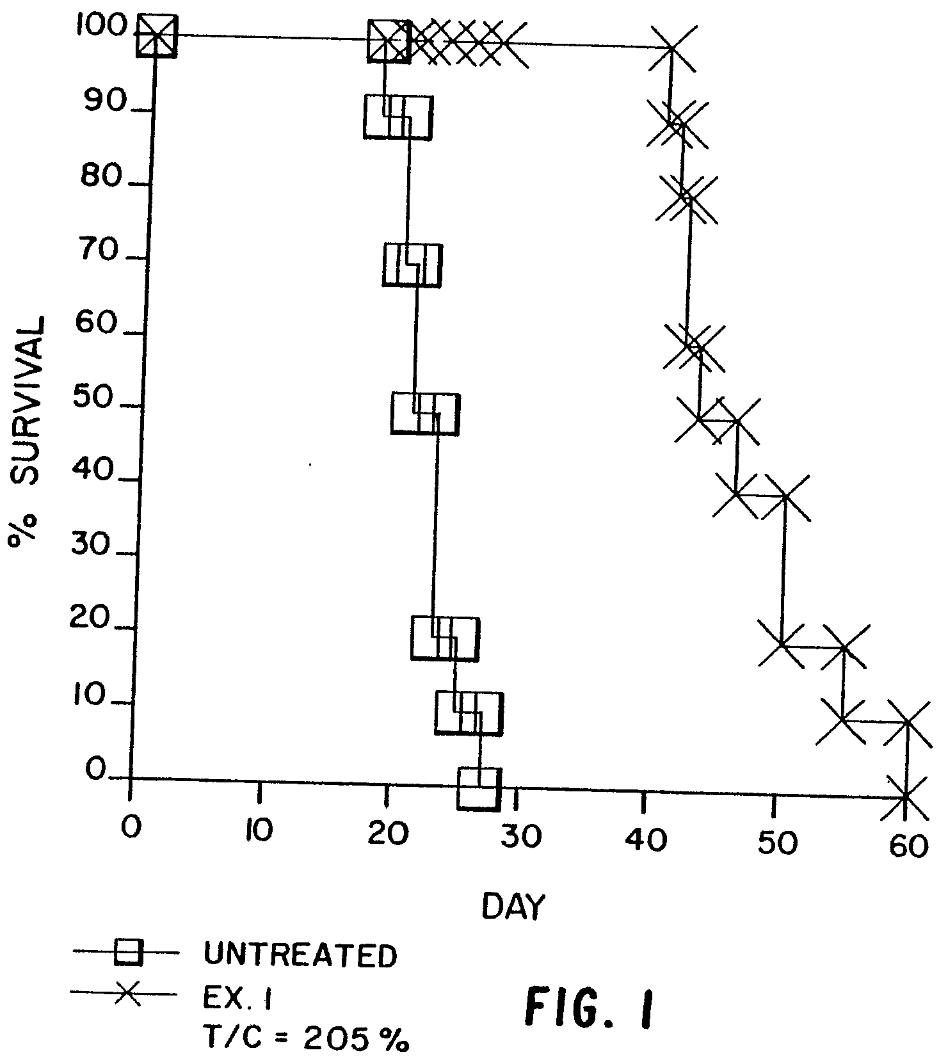
% SURVIVAL
100
90
80
70
60
50
40
30
20
10
0
0
10
20
30
40
50
60
DAY
UNTREATED
EX. 1
T/C = 205 %

FIG. 1

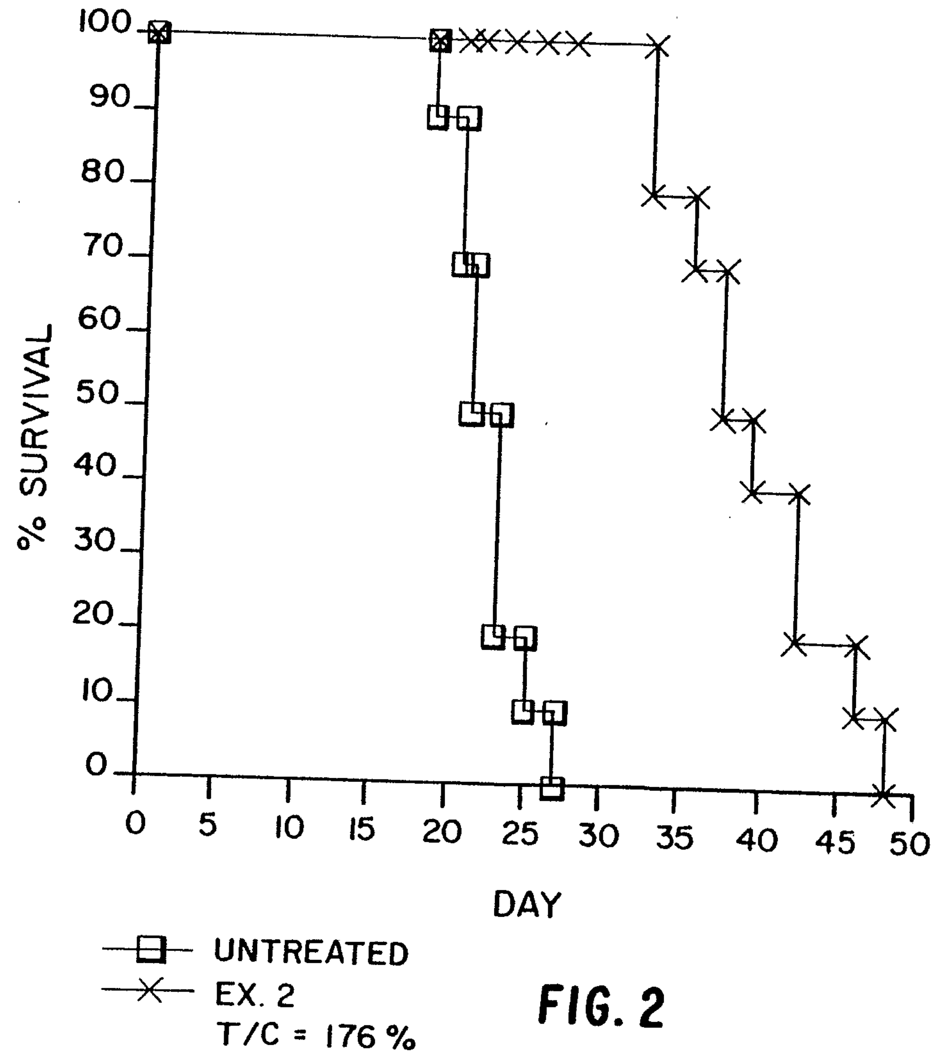
% SURVIVAL
100
90
80
70
60
50
40
30
20
10
0
0
5
10
15
20
25
30
35
40
45
50
DAY
UNTREATED
EX. 2
T/C = 176 %

FIG. 2

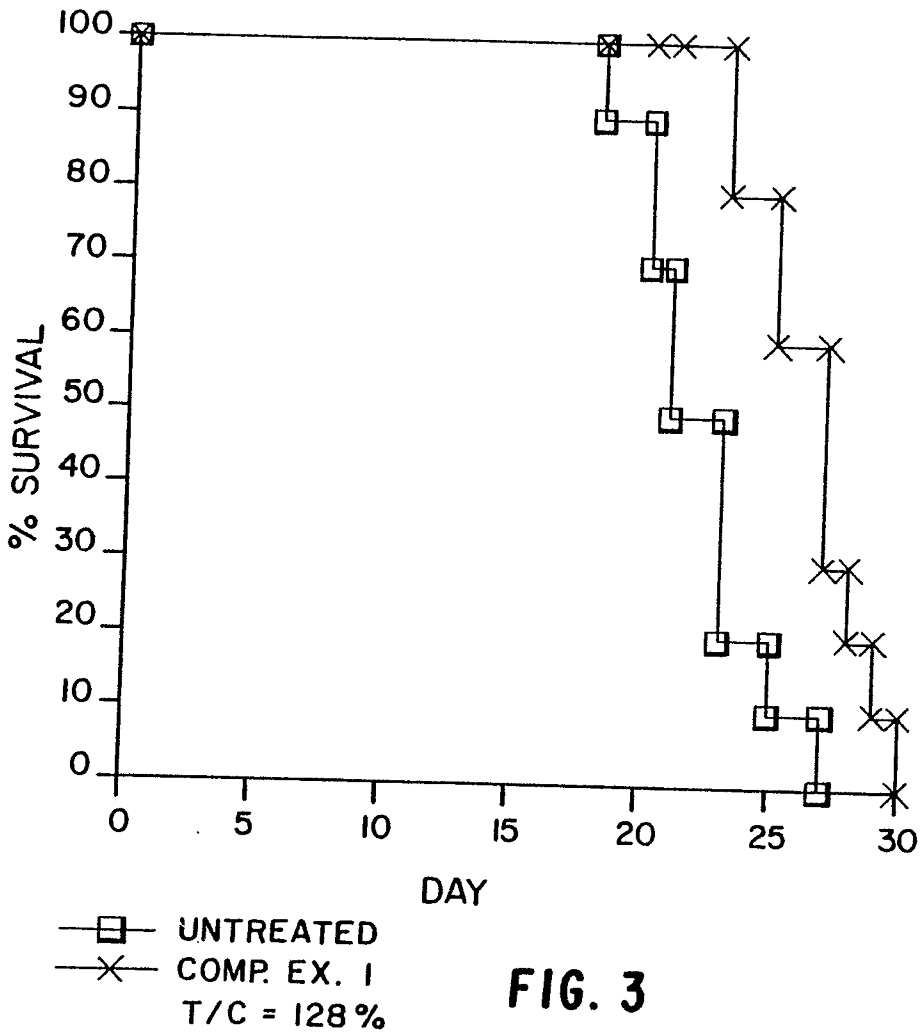
100
90
80
70
60
50
40
30
20
10
0
% SURVIVAL
0
5
10
15
20
25
30
DAY
UNTREATED
COMP. EX. 1
T/C = 128 %

FIG. 3

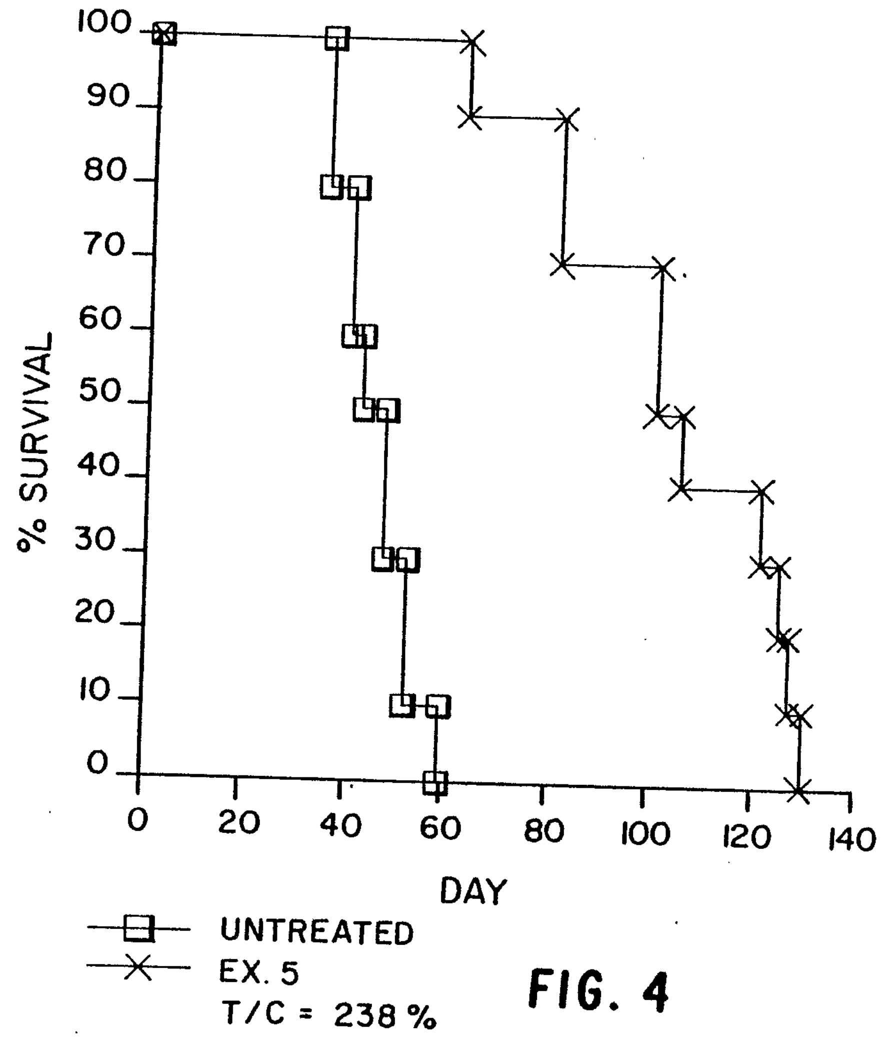
100
90
80
70
60
50
40
30
20
10
0
% SURVIVAL
0
20
40
60
80
100
120
140
DAY
UNTREATED
EX. 5
T/C = 238 %

FIG. 4

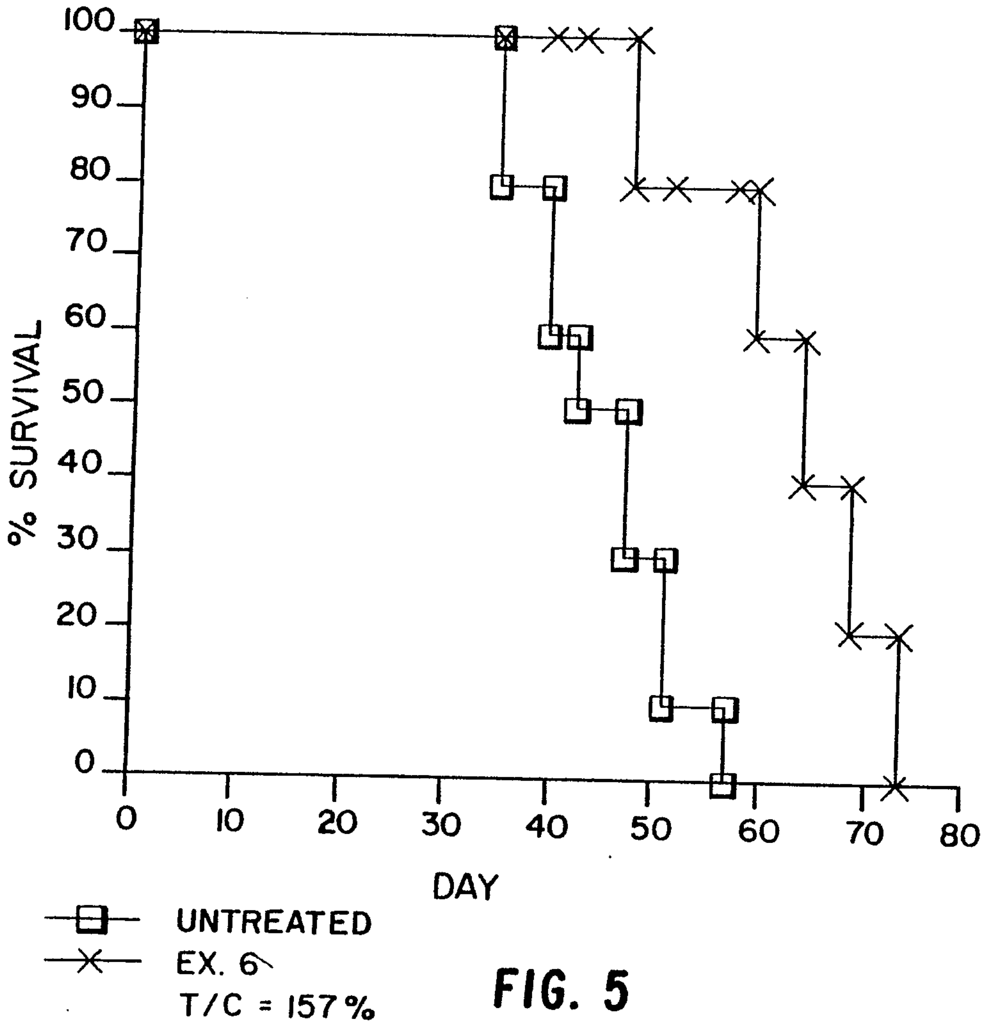
% SURVIVAL
100
90
80
70
60
50
40
30
20
10
0
0
10
20
30
40
50
60
70
80
DAY
UNTREATED
EX. 6
T/C = 157 %

FIG. 5

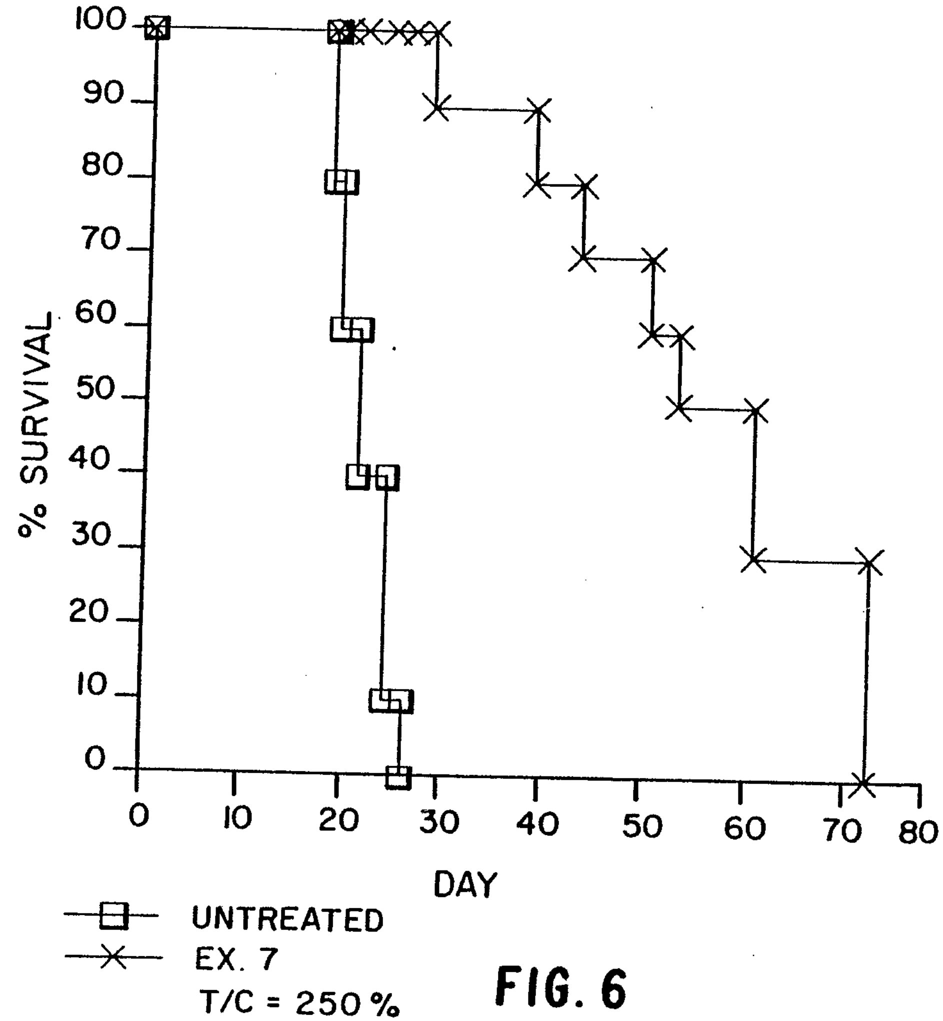
% SURVIVAL
100
90
80
70
60
50
40
30
20
10
0
0
10
20
30
40
50
60
70
80
DAY
UNTREATED
EX. 7
T/C = 250 %

FIG. 6

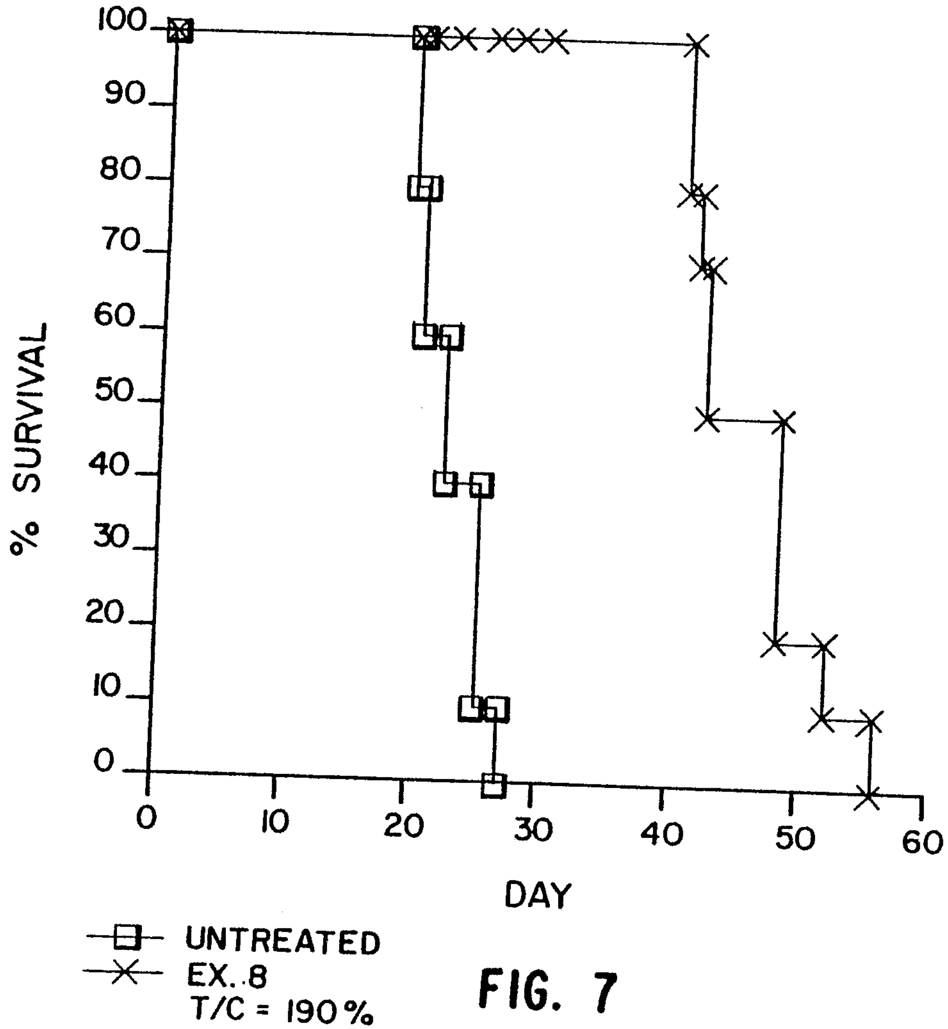

% SURVIVAL
100
90
80
70
60
50
40
30
20
10
0
0
10
20
30
40
50
60
DAY
UNTREATED
EX..8
T/C = 190%


FIG. 7

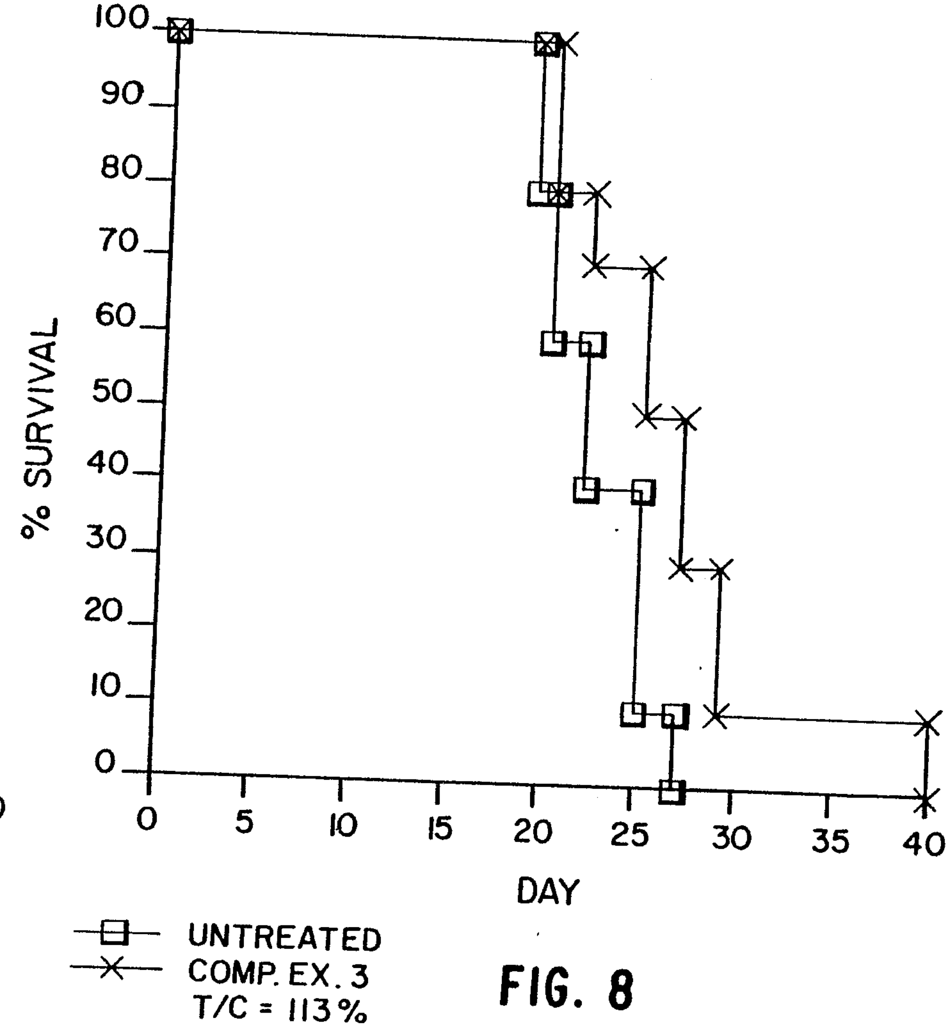

% SURVIVAL
100
90
80
70
60
50
40
30
20
10
0
0
5
10
15
20
25
30
35
40
DAY
UNTREATED
COMP. EX. 3
T/C = 113%


FIG. 8